(19) Europäisches Patentamt
European Patent Office
Office européen des brevets

(11) Publication number: **0 120 094**
**B1**

(12) # EUROPEAN PATENT SPECIFICATION

(45) Date of publication of patent specification: **19.04.89**

(51) Int. Cl.⁴: **C 07 D 513/04**

(21) Application number: **83903008.7**

(22) Date of filing: **24.09.83**

(86) International application number: **PCT/JP83/00315**

(87) International publication number: **WO 84/01152 29.03.84 Gazette 84/09**

(54) ## AZETIDINONE COMPOUNDS.

(30) Priority: **24.09.82 JP 167051/82**

(43) Date of publication of application: **03.10.84 Bulletin 84/40**

(45) Publication of the grant of the patent: **19.04.89 Bulletin 89/16**

(84) Designated Contracting States: **DE FR GB**

(56) References cited:
**EP-A-0 117 875**
**GB-A-1 472 868**
**GB-A-2 101 986**
**JP-A-51 043 791**
**JP-A-55 147 293**
**JP-A-57 059 896**
**JP-A-57 059 897**
**US-A-4 172 078**

**The file contains technical information submitted after the application was filed and not included in this specification**

(73) Proprietor: **OTSUKA KAGAKU YAKUHIN KABUSHIKI KAISHA**
**10, Bungomachi Higashi-ku**
**Osaka-shi Osaka 540 (JP)**

(72) Inventor: **TORII, Shigeru**
**4-4-18, Sanyo-Danchi Sanyocho**
**Akaiwa-gun Okayama 709-08 (JP)**
Inventor: **TANAKA, Hideo**
**1-2, Tsushimanaka Okayama-shi**
**Okayama 700 (JP)**
Inventor: **NOGAMI, Junzo**
**10-30-10, Tsushimahigashi 2-chome**
**Okayama-shi Okayama 700 (JP)**
Inventor: **SASAOKA, Michio**
**23-4, Aza Nakasu Nakamura Kitajimacho**
**Itano-gun Tokushima 771-02 (JP)**
Inventor: **SAITO, Norio**
**40-38, Aza Nakasao Shinkirai Kitajimacho**
**Itano-gun Tokushima 771-02 (JP)**
Inventor: **SHIROI, Takashi**
**72-2, Aza Motosu Nakamura Kitajimacho**
**Itano-gun Tokushima 771-02 (JP)**

(74) Representative: **Patentanwälte Dr. Solf & Zapf**
**Zeppelinstrasse 53**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Technical Field and Disclosure of the Invention

The present invention relates to novel azetidinone compounds represented by the formula

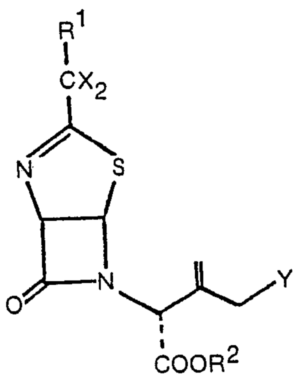

(I)

wherein $R^1$, $R^2$, X and Y are as defined below, which are useful as intermediates for synthesizing cephalosporin-type antibiotics.

In EP—A—117 875 and GB—A—2 101 986 azetidinone compounds having a structure similar to that of formula (I) are disclosed wherein X represents hydrogen and Y represents I, respectively.

From US—A—4 077 970 and EP—A—19 387 it has been already known that azetidinone derivatives having another, but similar structure are useful as intermediates in the synthesis of cephalosporins.

The object of the present invention is to find new azetidinone derivatives which are better suited to synthesize cephalosporin-type antibiotics.

It has now been found that the object of the invention can be achieved by novel azetidinone compounds represented by the general formula

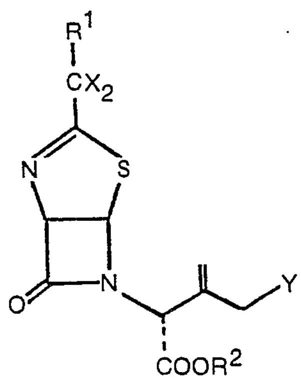

(I)

wherein

$R^1$ represents a phenyl optionally substituted with $CH_3$, Cl, Br, $NO_2$ or $CH_3O$,

$R^2$ represents a carboxyl-protecting group,

X represents a chlorine atom and

Y represents $—ONO_2$, —OH,

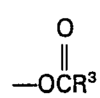

(wherein $R^3$ is a $C_1—C_4$ alkyl group or $—OR^5$ wherein $R^5$ is a halogen-containing $C_1—C_4$ alkyl group),

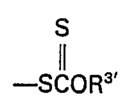

(wherein $R^{3'}$ is a $C_1—C_4$ alkyl group),

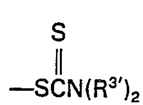

(wherein $R^{3'}$ is a $C_1$—$C_4$ alkyl group or —$SR^4$ (wherein $R^4$ is a 5-membered aromatic heterocyclic group containing sulfur and/or nitrogen atom or atoms as the heteroatom and optionally substituted with phenyl or methyl).

The present azetidinone derivatives of the above formula (I) are useful as intermediates for synthesizing cephalosporin-type antibiotics by using a simple process. By suitable selecting the substituents $R^3$ and $R^4$ in the above formula (I) it is possible to prepare new cephalosporin compounds which have a new acyl group.

According to a preferred embodiment of the present invention $R^1$ in formula (I) represents phenyl, tolyl, xylyl, p-chlorophenyl, p-bromophenyl, p-nitrophenyl or p-methoxyphenyl, in particular phenyl.

According to a further preferred embodiment of the present invention $R^2$ in formula (I) is a methyl group or a benzyl group.

$R^4$ in general formula (I) preferably represents 5-methyl-1,3,4-thiadiazole-2-yl, 1-methyl-1,2,3,4-tetrazole-5-yl, 1-phenyl-1,2,3,4-tetrazole-2-yl, 1,3,4-thiadiazole-2-yl or 1,3-thiazole-2-yl, in particular 1-methyl-1,2,3,4-tetrazole-5-yl.

Illustrative of carboxyl-protecting groups represented by $R^2$ are methyl, benzyl, diphenyl-methyl, p-nitrophenyl and trichloroethyl.

Examples of the $C_1$—$C_4$ alkyl groups represented by $R^3$ are those having 1 to 4 carbon atoms such as methyl, ethyl, propyl, isopropyl and butyl.

The halogen-containing $C_1$—$C_4$ alkyl groups represented by $R^5$ include, for example, 2-chloroethyl, 2-bromoethyl, 2,2-dichloroethyl, 2,2'-dibromoethyl, 2,2,2-trichloroethyl and 2,2,2-tribromoethyl.

The compound (I) of the present invention can be prepared by using a compound of the formula (IV)

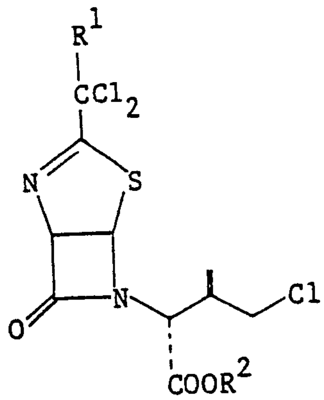

(IV)

wherein $R^1$ and $R^2$ are as defined above as the starting material by various processes as stated hereinafter.

The compound (IV) can be prepared by the conventional method, for example, that reported in Tetrahedron Lett. *22*, 3193 (1981).

(1) The compound of the present invention represented by the formula

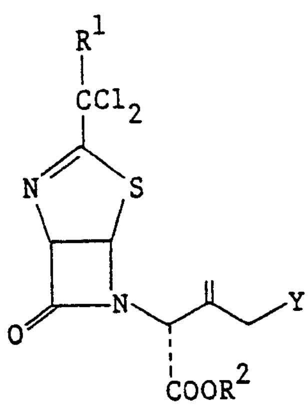

wherein $R^1$ and $R^2$ are as defined above and Y represents

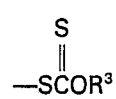

(in which $R^3$ is as defined above) can be prepared by reacting the starting material (IV) with a nucleophilic reagent of the formula

M—Y

wherein Y represents

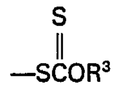

(in which $R^3$ is as defined above) and M represents sodium or potassium atom in an organic solvent.

Useful organic solvents can be any of those capable of dissolving the starting compound (IV) and the nucleophilic reagent, such as acetone, methyl ethyl ketone, methylisobutyl ketone; methyl formate, ethyl formate, methyl acetate, or ethyl acetate; diethyl ether, tetrahydrofuran or dioxane; nitromethane or nitroethane; acetonitrile or propionitrile; methanol or ethanol; dimethylformamide or dimethylacetamides; and dimethylsulfoxide; among which acetone and dimethylsulfoxide are preferred.

The amount of the organic solvent is not particularly limited but is usually 1 to 200 times, preferably 2 to 50 times, the weight of the compound (IV).

The ratio between the compound (IV) and the nucleophilic reagent can be suitably determined over a wide range. Usually about 1 to about 5 moles, preferably about 1 to about 3 moles, of the latter is used per mole of the former.

The reaction is carried out at a temperature of usually about −10 to about 70°C, preferably about 10 to about 55°C and is completed generally in about 0,5 to about 4 hours.

(2) The present compound of the formula

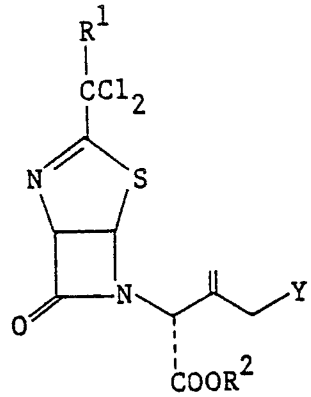

wherein $R^1$ and $R^2$ are as defined above and Y represents

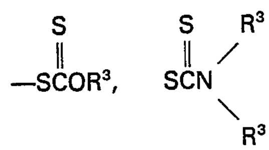

,

or $—SR^4$ (in which $R^3$ and $R^4$ are as defined above) can be prepared by reacting the thiazolino-azetidinone compound of the following formula produced by the process described above in (1)

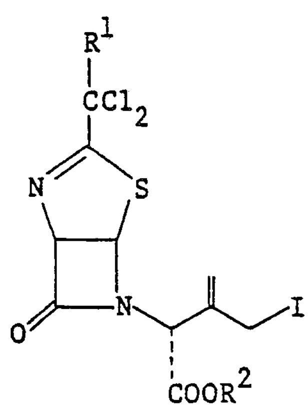

(VI)

wherein $R^1$ and $R^2$ are as defined above with a nucleophilic reagent of the formula

M—Y

wherein M represents sodium or potassium atom and Y represents

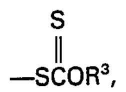

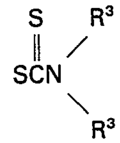

or $-SR^4$ (in which $R^3$ and $R^4$ are as defined above) in an organic solvent to selectively subject only the iodine atom to nucleophilic substitution.

Useful organic solvents can be any of those capable of dissolving the compound (VI) and the nucleophilic reagent. Extensive use is made of the organic solvents to be used in the reaction between the compound (IV) and the nucleophilic reagent. Preferable examples of useful solvents are acetone, dimethylsulfoxide or dimethylformamide among which acetone is more preferable.

The amount of the organic solvent is not particularly limited, but is usually 1 to 200 times, preferably 2 to 50 times, the weight of the compound (VI).

The ratio between the compound (VI) and the nucleophilic reagent can be suitably determined over a wide range, but the latter is used in an amount of usually 1 to 3 moles, preferably 2 to 1,5 moles, of the former.

The reaction is conducted at a temperature of usually about −10 to about 40°C, preferably around room temperature, and is completed generally in about 0,2 to about 2 hours.

(3) The compound of the present invention represented by the formula

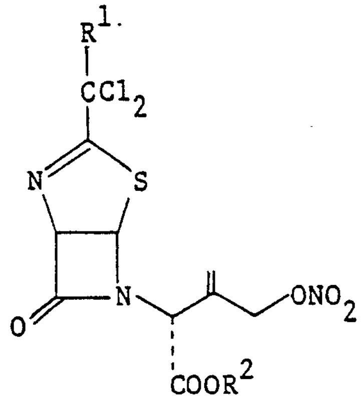

wherein $R^1$ and $R^2$ are as defined above can be prepared by reacting the compound of the following formula (VI) produced by the process stated above in (1):

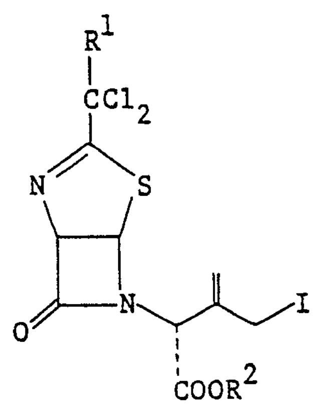

(VI)

wherein $R^1$ and $R^2$ are as defined above with $NaNO_3$ or $KNO_3$ in an organic solvent such as dimethylsulfoxide, dimethylformamide or hexamethylphosphoamide.

The organic solvent is used in an amount of usually 1 to 200 times, preferably 2 to 50 times, the weight of the compound (VI).

The ratio between the compound (VI) and $NaNO_3$ or $KNO_3$ can be suitably determined over a wide range. Usually about 1 to about 10 moles, preferably about 1 to about 5 moles, of the latter is used per mole of the former.

The reaction is effected at a temperature of usually about 0 to about 100°C, preferably about 40 to about 60°C.

Preferably the reaction is performed at reduced pressure of about 4 kPa (30 mm Hg) to about 10,7 kPa (80 mm Hg) in the presence of methyl methanesulfonate or methyl toluenesulfonate in order to remove the NaI or KI produced as a by-product with the progress of the reaction. The reaction is completed usually in about 2 to about 6 hours.

The compound of the present invention represented by the formula

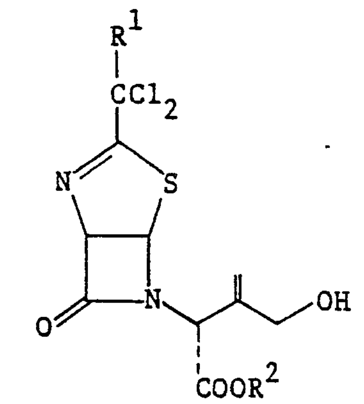

wherein $R^1$ and $R^2$ is as defined above can be prepared by subjecting the compound (VI) to the action of an acid catalyst in hydrous dimethylsulfoxide, hydrous dimethylformamide or hydrous hexamethylphosphoamide to selectively replace only the iodine atom with —OH.

The amount of the water contained in the solvent is usually about 1 to about 100 moles, preferably about 1 to about 50 moles, per mole of the compound (VI).

The solvent is employed in an amount of usually 1 to 200 times, preferably 2 to 50 times, the weight of the compound (VI).

Useful acid catalysts include p-toluenesulfonic acid, methanesulfonic acid, trifluoroacetic acid, trichloroacetic acid and dichloroacetic acid.

The acid catalyst is used in an amount of usually about 0,1 to about 6 moles, preferably about 0,1 to about 3 moles, per mole of the compound (VI).

The reaction is conducted at a temperature of usually 20 to 120°C, preferably 40 to 80°C, and is completed usually in about 1 to about 5 hours.

The compound (wherein Y = —OH) thus obtained is reacted with a lower carboxylic acid anhydride or lower carboxylic acid halide having 1 to 5 carbon atoms or a compound represented by the formula

$$X-\overset{O}{\overset{\|}{C}}OR^5 \qquad (XI)$$

wherein $R^5$ represents a halogen-containing $C_1$—$C_4$ alkyl group in the presence of pyridine, polyvinyl pyridine, molecular sieve or like acid-trapping agent, thereby giving the compound of the present invention represented by the formula

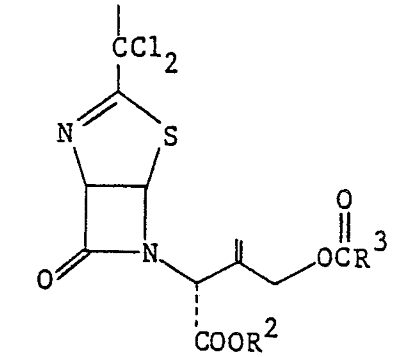

wherein $R^1$, $R^2$ and $R^3$ are as defined above.

The reaction is carried out in an organic solvent inert to the reaction.

Examples of useful solvents are acetone, methyl ethyl ketone and methyl-iso-butyl ketone; diethyl ether, diisopropyl ether, dibutyl ether, tetrahydrofuran and dioxan; dichloromethane, dibromomethane, chloroform, bromoform, carbon tetrachloride, dichloroethane, dibromoethane and trichloroethane; benzene, toluene and xylene; methyl formate, ethyl formate, methyl acetate, ethyl acetate and methyl propionate; acetonitrile and propionitrile; nitromethane, nitroethane and nitropropane.

The organic solvent is used in an amount of usually 1 to 200 times, preferably 2 to 50 times, the weight of the compound (XI).

The ratio between the compound (in which Y = —OH) and the lower carboxylic acid anhydride or lower carboxylic acid halide or compound (XI) can be selected over a wide range. Usually the latter is used in an amount of usually about 1 to about 10 moles, preferably about 1 to about 5 moles, per mole of the former.

The reaction is conducted preferably at a relatively low temperature in the range of about −50 to about 10°C and is completed usually in about 1 to about 10 hours.

Example 1

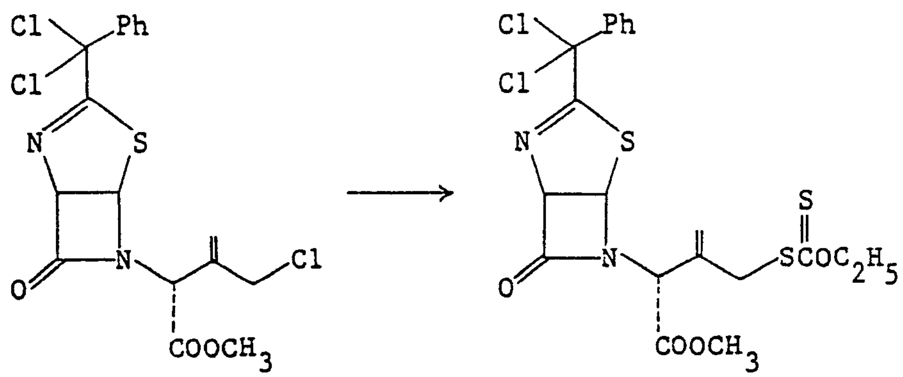

A 4 ml quantity of acetone was added to 249.2 mg of methyl 2-(3-phenyldichloromethyl-7-oxo-4-thia-2,6-diazabicyclo[3,2,0]hepto-2-ene-6-yl)-3-chloromethyl-3-butenate to obtain a uniform solution. To the solution was added 248.5 mg of

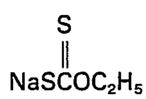

and the mixture was subjected to reaction at room temperature for 1 hour. The reaction mixture was diluted with 20 ml of diethyl ether and the dilution was washed with a saturated aqueous solution of sodium chloride and dried over $Na_2SO_4$. The solvent was distilled off at reduced pressure. The resulting pale yellow oily residue was subjected to silica gel column chromatography using benzene-ethyl acetate (10:1), giving 268.5 mg of methyl 2-(3-phenyldichloromethyl-7-oxo-4-thia-2,6-diazabicyclo[3,2,0]hepto-2-ene-6-yl)-3-ethoxythiocarbonylthiomethyl-3-butenate in 90% yield.

NMR (δ, $CDCl_3$) 1.39 (t, 3H, 7Hz), 3.70 (s, 3H),
4.63 (q, 2H, 7Hz), 5.04 (s, 1H),
5.11 (s, 1H), 5.39 (s, 1H), 6.02 (s, 2H),
7.20—7.55 (m, 3H), 7.55—7.85 (m, 2H)

Example 2

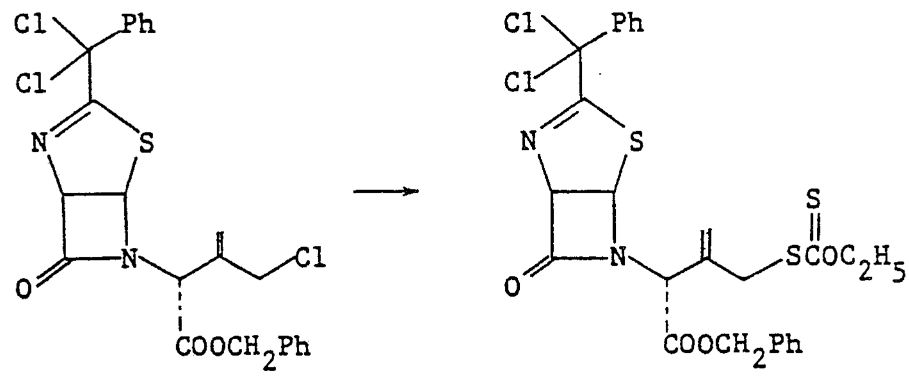

Using as the starting material 50.0 mg of benzyl 2-(3-phenyldichloromethyl-7-oxo-4-thia-2,6-diazabicyclo[3,2,0]hepto-2-ene-6-yl)-3-chloromethyl-3-butenate, the same reaction and treatment as in Example 1 were conducted, giving 49.5 mg of benzyl 2-(3-phenyldichloromethyl-7-oxo-4-thia-2,6-diazabicyclo[3,2,0]-hepto-2-ene-6-yl)-3-ethoxythiocarbonylthiomethyl-3-butenate in 85% yield.

IR (neat) 1775, 1735 $cm^{-1}$
NMR (δ, $CDCl_3$) 1.35 (t, 3H, 7Hz), 3.70 (s, 2H),
4.59 (q, 2H, 7Hz), 5.00 (s, 1H),
5.09 (s, 1H), 5.15 (s, 2H), 5.32 (s, 1H),
6.00 (s, 2H), 7.25—7.60 (m, 8H),
7.60—7.85 (m, 2H)

Example 3

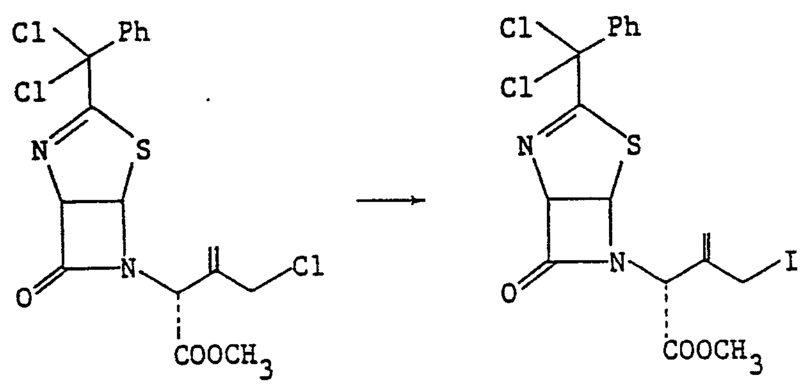

A 1 ml quantity of acetone was added to 30.7 mg of methyl 2-(3-phenyldichloromethyl-7-oxo-4-thia-2,6-diazabicyclo[3,2,0]hepto-2-ene-6-yl)-3-chloromethyl-3-butenate to obtain a uniform solution. To the solution was added 22.0 mg of NaI and the mixture was subjected to reaction for 3 hours while being heated to 55°C. The reaction mixture was cooled to room temperature and was diluted with ethyl acetate. The dilution was washed with an aqueous solution of $Na_2S_2O_3$ and then with a saturated aqueous solution of sodium chloride and dried over $Na_2SO_4$. The solvent was distilled off at reduced pressure. The resulting pale yellow oily residue was subjected to silica gel column chromatography using benzene-ethyl acetate (10:1), giving 30.0 mg of methyl 2-(3-phenyldichloromethyl-7-oxo-4-thia-2,6-diazabicyclo[3,2,0]hepto-2-ene-6-yl)-3-iodomethyl-3-butenate in 81% yield.

IR ($CHCl_3$) 1780, 1745 $cm^{-1}$
NMR (δ, $CDCl_3$) 3.55 and 3.69 (ABq, 2H, 10Hz),
3.75 (s, 3H), 5.05 (s, 1H),
5.24 (s, 1H), 5.46 (s, 1H),
6.04 and 6.09 (ABq, 2H, 4Hz),
7.20—7.60 (m, 3H), 7.60—7.90 (m, 2H)

Example 4

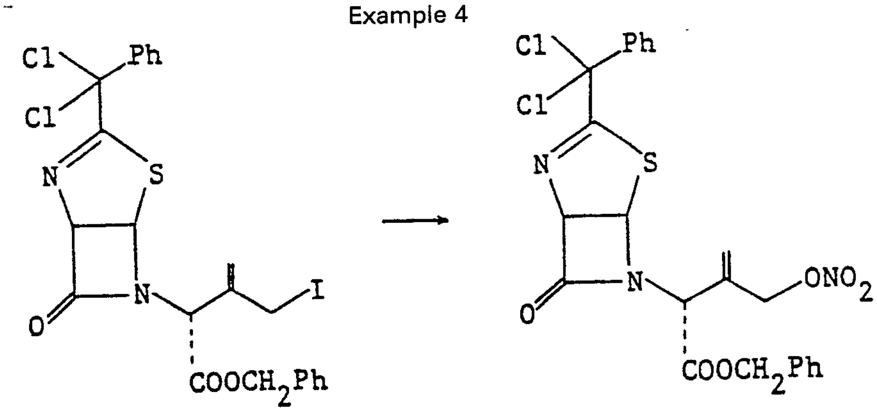

A 0.4 ml quantity of dimethylsulfoxide was added to 25.3 mg of benzyl 2-(3-phenyldichloromethyl-7-oxo-4-thia-2,6-diazabicyclo[3,2,0]hepto-2-ene-6-yl)-3-iodomethyl-3-butenate to obtain a uniform solution. To the solution were added 14.3 mg of $NaNO_3$ and 7.0 mg of methyl methanesulfonate to dissolve therein. The reaction system was heated to 65°C and subjected to reaction for 2 hours and 40 minutes while being maintained at reduced pressure of 55 mm Hg with use of a water-jet pump. The reaction mixture was left to stand to cool to room temperature and an aqueous solution of $Na_2S_2O_3$ was added, followed by vigorous agitation. The reaction mixture was extracted with ethyl acetate, and the organic layer was washed with a saturated aqueous solution of sodium chloride and was dried over $Na_2SO_4$. The solvent was distilled off at reduced pressure. The resulting yellow oily residue was subjected to silica gel column chromatography using benzene-ethyl acetate (80:1) and then benzene ethyl acetate (10:1), 18.1 mg of benzyl 2-(3-phenyldichloromethyl-7-oxo-4-thia-2,6-diazabicyclo[3,2,0]hepto-2-ene-6-yl)-3-nitroxymethyl-3-butenate in 80% yield.

IR (neat) 1775, 1740, 1635, 1275 $cm^{-1}$
NMR (δ, $CDCl_3$) 4.74 (s, 2H), 5.04 (s, 1H), 5.18 (s, 2H),
5.23 (s, 1H), 5.44 (s, 1H),
6.02 and 6.06 (ABq, 2H, 4Hz),
7.32 (s, 5H), 7.15—7.55 (m, 3H),
7.55—7.85 (m, 2H)

Example 5

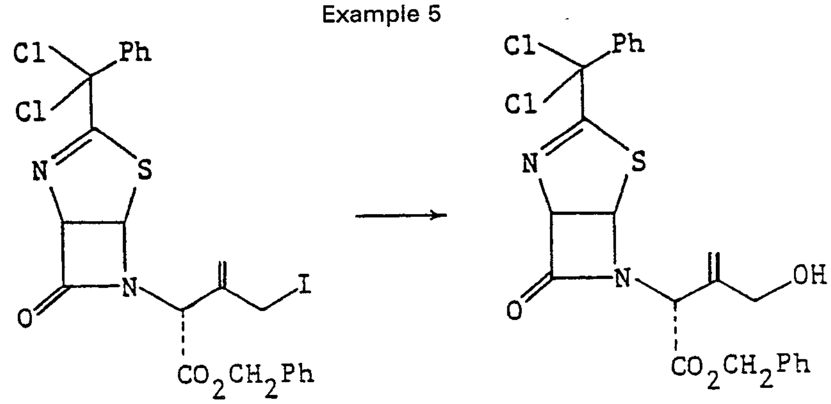

A 0.3 ml quantity of dimethylsulfoxide was added to 35.3 mg of benzyl 2-(3-phenyldichloromethyl-7-oxo-4-thia-2,6-diazabicyclo[3,2,0]hepto-2-ene-6-yl)-3-iodomethyl-3-butenate to give a uniform solution. To the solution was added 14.0 mg of p-toluenesulfonic acid to dissolve therein. The solution was heated to 50°C to continue reaction for 2 hours. The reaction mixture was allowed to stand to cool to room temperature. An aqueous solution of $Na_2S_2O_3$ was added thereto and the mixture was vigorously stirred, followed by extraction with ethyl acetate. The organic layer was washed with a saturated aqueous solution of sodium chloride, and was dried over $Na_2SO_4$ and the solvent was distilled off at reduced pressure. The resulting yellow oily residue was subjected to silica gel column chromatography using benzene-ethyl acetate (10:1), to afford 22.6 mg of benzyl 2-(3-phenyldichloromethyl-7-oxo-4-thia-2,6-diazabicyclo[3,2,0]-hepto-2-ene-6-yl)-3-hydroxymethyl-3-butenate in 78% yield.

IR (neat) 3440, 1780, 1745, 1160 $cm^{-1}$
NMR (δ, $CDCl_3$) 1.75 (bs, 1H), 4.00 (s, 2H),
5.01 (s, 1H), 5.11 (s, 1H), 5.19 (s, 2H),
5.30 (s, 1H), 6.05 (s, 2H), 7.36 (s, 5H),
7.20—7.60 (m, 3H), 7.60—7.90 (m, 2H)

Example 6

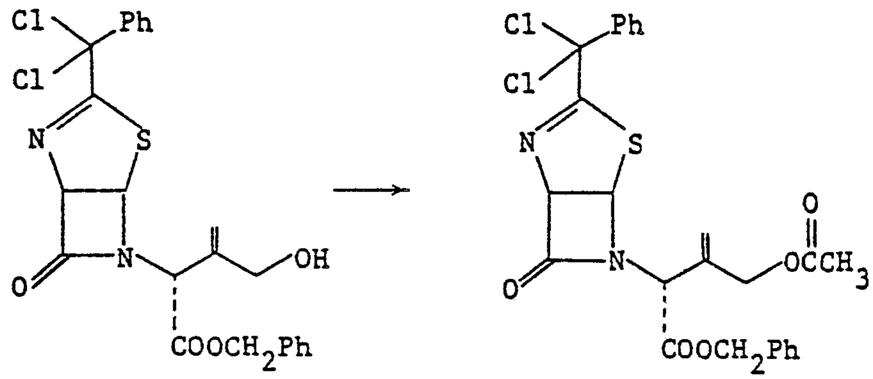

The same procedure was repeated by using as the starting material 26.0 mg of benzyl 2 - (3 - phenyldichloromethyl - 7 - oxo - 4 - thia - 2,6 - diazabicyclo[3,2,0]hepto - 2 - ene - 6 - yl) - 3 - hydroxymethyl - 3 - butenate, producing 27.0 mg of benzyl 2 - (3 - phenyldichloromethyl - 7 - oxo - 4 - thia - 2,6 - diazabicyclo[3,2,0]hepto - 2 - ene - 6 - yl) - 3 - acetoxymethyl - 3 - butenate in 96% yield.

IR (neat) 1770, 1740, 1730, 1220 $cm^{-1}$
NMR (δ, $CDCl_3$) 2.02 (s, 3H), 4.47 (s, 2H), 5.09 (s, 2H),
5.16 (s, 2H), 5.30 (s, 1H), 6.01 (s, 2H),
7.31 (s, 5H), 7.20—7.60 (m, 3H),
7.60—7.90 (m, 2H)

Example 7

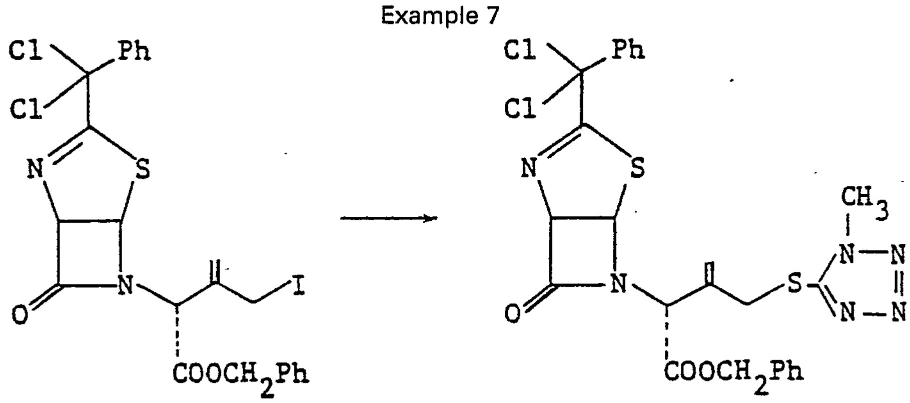

A 0.5 ml quantity of acetone was added to 43.8 mg of benzyl 2-(3-phenyldichloromethyl-7-oxo-4-thia-2,6-diazabicyclo[3,2,0]hepto-2-ene-6-yl)-3-iodomethyl-3-butenate to obtain a uniform solution. To the solution was added 10.5 mg of

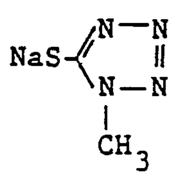

and the mixture was stirred at room temperature for 35 minutes to undergo reaction. The reaction mixture was diluted with 3 ml of ethyl acetate and the dilution was washed successively with an aqueous solution of $Na_2S_2O_3$ and with a saturated aqueous solution of sodium chloride and dried over $Na_2SO_4$. The solvent was distilled off at reduced pressure. The resulting pale yellow oily residue was subjected to silica gel column chromatography using benzene-ethyl acetate (20:1), giving 36.2 mg of benzyl 2 - (3 - phenyldichloromethyl - 7 - oxo - 4 - thia - 2,6 - diazabicyclo[3,2,0]hepto - 2 - ene - 6 - yl) - 3 - (1 - methyl - 1,2,3,4 - tetrazole - 5 - yl - thiomethyl) - 3 - butenate in 89% yield.

IR (neat) 1775, 1740 $cm^{-1}$
NMR (δ, $CDCl_3$) 3.45 and 3.98 (ABq, 2H, 14Hz),
3.73 (s, 3H), 4.94 (s, 1H), 5.10 (s, 1H),
5.19 (s, 1H), 5.33 (s, 1H),
6.02 and 6.09 (ABq, 2H, 4Hz),
7.33 (s, 5H), 7.10—7.60 (m, 3H),
7.60—7.90 (m, 2H)

Example 8

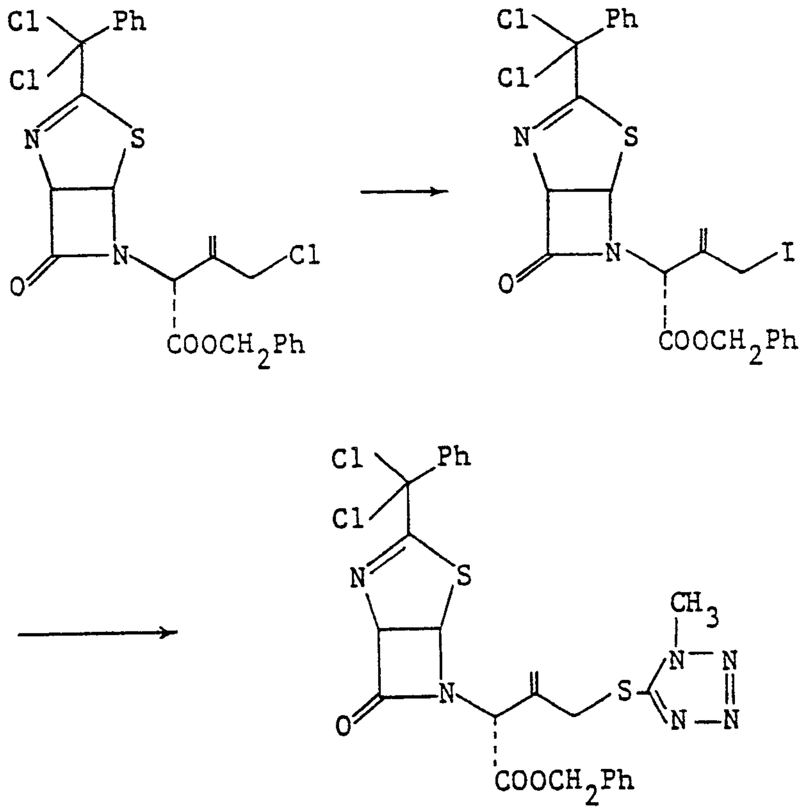

A 0.5 ml quantity of acetone was added to 37.1 mg of benzyl 2-(3-phenyldichloromethyl-7-oxo-4-thia-2,6-diazabicyclo[3,2,0]hepto-2-ene-6-yl)-3-chloromethyl-3-butenate to obtain a uniform solution. To the solution was added 21.8 mg of NaI and the mixture was subjected to reaction for 2 hours while being heated to 53°C. The reaction mixture was allowed to stand to cool to room temperature and 11.1 mg of

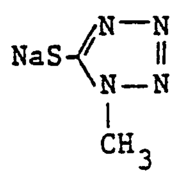

was added. The mixture was stirred at room temperature for 20 minutes to continue reaction. The reaction mixture was treated in the same manner as above to 36.5 mg of benzyl 2-(3-phenyldichloromethyl-7-oxo-4-thia-2,6-diazabicyclo[3,2,0]hepto-2-ene-6-yl)-3-(1-methyl-1,2,3,4-tetrazole-5-yl-thiomethyl)-3-butenate in 85% yield. The compound thus obtained was chemically analyzed with the result identical with that of the compound produced in Example 7.

Example 9

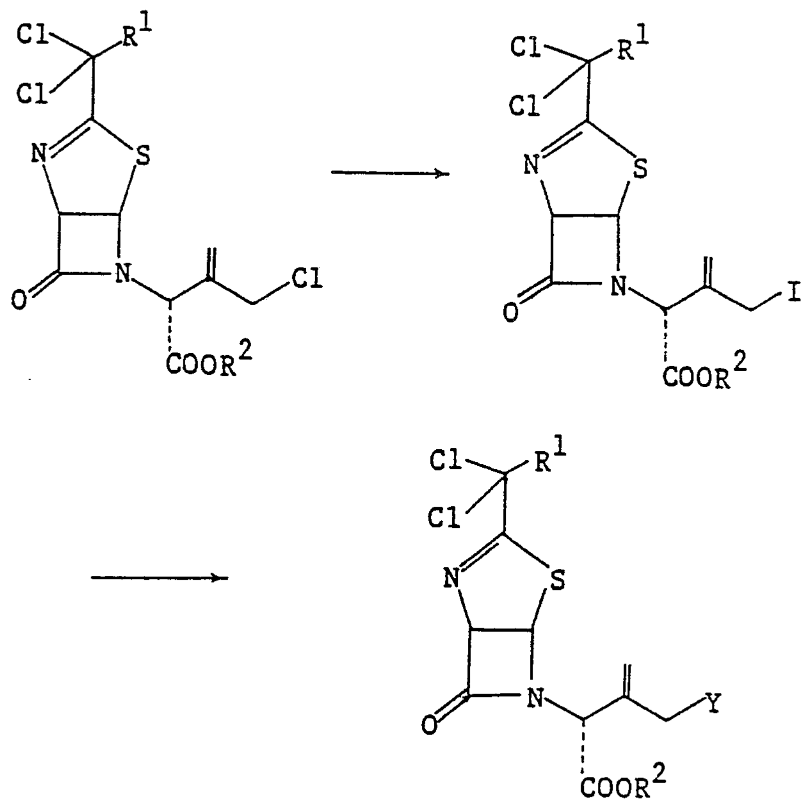

The compounds as shown below in a table were obtained by following the procedure of Example 8. The table also indicates the yields and the results of chemical analysis.

| $R^1$ | $R^2$ | Y | Yield (%) | IR ($cm^{-1}$) | NMR (δ, $CDCl_3$) |
|---|---|---|---|---|---|
| Ph | $PhCH_2$ | 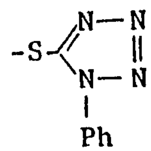 | 95 | 1775<br>1740 | 3.55 and 4.08 (ABq, 2H, 14Hz), 5.03 (s, 1H), 5.09 (s, 2H), 5.14 (s, 1H), 5.50 (s, 1H), 5.99 and 6.02 (ABq, 2H, 4Hz), 7.15-7.85 (m, 15H) |
| Ph | $PhCH_2$ | 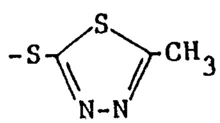 | 79 | 1770<br>1735 | 2.63 (s, 3H), 3.53 and 3.92 (ABq, 2H, 14Hz), 5.00 (s, 1H), 5.14 (s, 2H), 5.22 (s, 1H), 5.33 (s, 1H), 6.01 and 6.04 (ABq, 2H, 4Hz), 7.20-7.55 (m, 3H), 7.32 (s, 5H), 7.55-7.85 (m, 2H) |
| Ph | $PhCH_2$ | 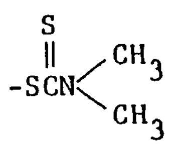 | 90 | 1775<br>1745 | 3.33 (bs, 3H), 3.45 (bs, 3H), 4.05 (s, 2H), 5.01 (s, 1H), 5.10 (s, 1H), 5.15 (s, 2H), 5.39 (s, 1H), 5.99 (s, 2H), 7.32 (s, 5H), 7.15-7.55 (m, 3H), 7.55-7.85 (m, 2H) |
| Ph | $PhCH_2$ | 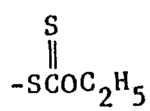 | 75 | 1775<br>1735 | The result is identical with that of the compound obtained in Example 2. |

## Claims

1. An azetidinone compound represented by the formula

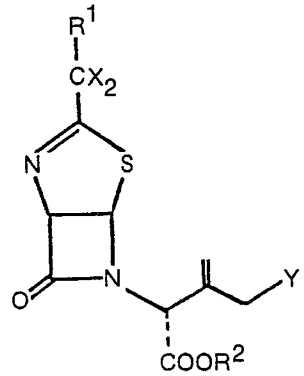

(I)

wherein

$R^1$ represents a phenyl optionally substituted with $CH_3$, Cl, Br, $NO_2$ or $CH_3O$,
$R^2$ represents a carboxyl-protecting group,
X represents a chlorine atom and
Y represents —$ONO_2$, —OH,

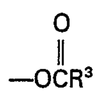

(wherein $R^3$ is a $C_1$—$C_4$ alkyl group or —$OR^5$ wherein $R^5$ is a halogen-containing $C_1$—$C_4$ alkyl group),

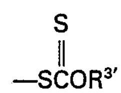

(wherein $R^{3'}$ is a $C_1$—$C_4$ alkyl group),

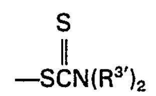

(wherein $R^{3'}$ is a $C_1$—$C_4$ alkyl group) or —$SR^4$ (wherein $R^4$ is a 5-membered aromatic heterocyclic group containing sulfur and/or nitrogen atom or atoms as the heteroatom and optionally substituted with phenyl or methyl.

2. An azetidinone compound as defined in claim 1 wherein $R^1$ is phenyl, tolyl, xylyl, p-chlorophenyl, p-bromophenyl, p-nitrophenyl or p-methoxyphenyl.

3. A compound as defined in claim 2, wherein $R^1$ is a phenyl group.

4. A compound as defined any one of claims 1—3, wherein $R^2$ is a methyl group or a benzyl group.

5. A compound as defined in any one of claims 1—4, wherein $R^4$ is 5-methyl-1,3,4-thiadiazole-2-yl, 1-methyl-1,2,3,4-tetrazole-5-yl, 1-phenyl-1,2,3,4-tetrazole-2-yl or 1,3,4-thiadiazole-2-yl or 1,3-thiazole-2-yl.

6. A compound as defined in any one of claims 1—5, wherein Y is as defined in claim 1 and $R^4$ is a 1-methyl-1,2,3,4-tetrazole-5-yl-group.

**Patentansprüche**

1. Azedidinon-Verbindung der Formel

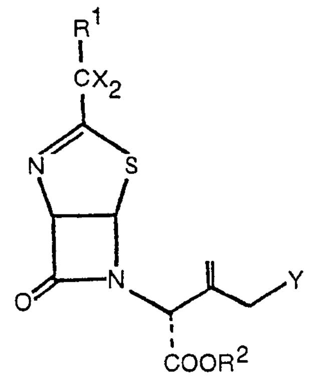

(I)

worin bedeuten:

$R^1$ eine Phenylgruppe, die ggf. substituiert ist durch $CH_3$, Cl, Br, $NO_2$ oder $CH_3O$,
$R^2$ eine Carboxyl-Schutzgruppe,
X ein Chloratom und
Y $-ONO_2$, $-OH$,

$$-\overset{\overset{O}{\|}}{C}R^3$$

(worin $R^3$ eine $C_1-C_4$-Alkylgruppe oder $-OR^5$ darstellt, worin $R^5$ für eine Halogen enthaltende $C_1-C_4$-Alkylgruppe steht),

$$-S\overset{\overset{S}{\|}}{C}OR^{3'}$$

(worin $R^{3'}$ eine $C_1-C_4$-Alkylgruppe darstellt),

$$-S\overset{\overset{S}{\|}}{C}N(R^{3'})_2$$

(worin $R^{3'}$ eine $C_1-C_4$-Alkylgruppe darstellt) oder $-SR^4$ (worin $R^4$ eine 5-gliedrige, aromatische, heterocyclische Gruppe darstellt, die ein oder mehrere Schwefel- und/oder Stickstoffatome als Heteroatome enthält und ggf. substituiert ist durch Phenyl oder Methyl.

2. Azetidinon-Verbindung nach Anspruch 1, worin $R^1$ Phenyl, Tolyl, Xylyl, p-Chlorophenyl, p-Bromophenyl, p-Nitrophenyl oder p-Methoxyphenyl bedeutet.

3. Verbindung nach Anspruch 2, worin $R^1$ eine Phenylgruppe bedeutet.

4. Verbindung nach einem der Ansprüche 1 bis 2, worin $R^2$ eine Methylgruppe oder eine Benzylgruppe bedeutet.

5. Verbindung nach einem der Ansprüche 1 bis 4, worin $R^4$ 5-Methyl-1,3,4-thiadiazol-2-yl, 1-Methyl-1,2,3,4-tetrazol-5-yl, 1-Phenyl-1,2,3,4-tetrazol-2-yl oder 1,3,4-Thiadiazol-2-yl oder 1,3-Thiazol-2-yl bedeutet.

6. Verbindung nach einem der Ansprüche 1 bis 5, worin Y wie in Anspruch 1 definiert ist und $R^4$ eine 1-Methyl-1,2,3,4-tetrazol-5-yl-Gruppe bedeutet.

**Revendications**

1. Composé d'azétidinone caractérisé en ce qu'il est représenté par la formule

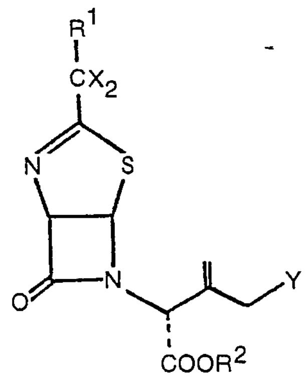

(I)

dans laquelle
$R^1$ représente un groupe phényle éventuellement substitué avec $CH_3$, Cl, Br, $NO_2$ ou $CH_3O$,
$R^2$ représente un groupe protégeant la fonction carboxy,
X représente un atome de chlore et
Y représente $-ONO_2$, $-OH$,

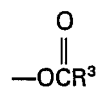

(où $R^3$ est un groupe alkyle en $C_{1-4}$ ou $-OR^5$ dans lequel $R^5$ est un groupe halo-alkyle en $C_{1-4}$)

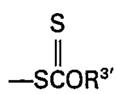

(où $R^{3'}$ est un groupe alkyle en $C_{1-4}$),

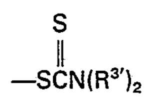

(dans lequel $R^{3'}$ est un groupe alkyle en $C_{1-4}$) ou $-SR^4$ (où $R^4$ est un groupe hétérocyclique aromatique à 5 chaînons contenant un ou des atomes de soufre et/ou d'azote en tant qu'hétéroatome et éventuellement substitué avec un groupe phényle ou méthyle.

2. Composé d'azétidinone suivant la revendication 1, caractérisé en ce que $R^1$ est un groupe phényle, tolyle, xylyle, p-chlorophényle, p-bromophényle, p-nitrophényle ou p-méthoxyphényle.

3. Composé suivant la revendication 2, caractérisé en ce que $R^1$ est un groupe phényle.

4. Composé suivant l'une quelconque des revendications 1 à 3, caractérisé en ce que $R^2$ est un groupe méthyle ou un groupe benzyle.

5. Composé suivant l'une quelconque des revendications 1 à 4, caractérisé en ce que $R^4$ est un groupe 5-méthyl-1,3,4-thiadiazole-2-yle, 1-méthyle-1,2,3,4-tétrazole-5-yle, 1-phényl-1,2,3,4-tétrazole-2-yle, 1,3,4-thiadiazole-2-yle ou 1,3-thiazole-2-yle.

6. Composé suivant l'une quelconque des revendications 1 à 5, caractérisé en ce que Y est tel que défini dans la revendication 1 et $R^4$ est un groupe 1-méthyl-1,2,3,4-tétrazole-5-yle.